# EUROPEAN PATENT APPLICATION

(11) **EP 1 060 677 A1**
(43) Date of publication of application: **20.12.2000**
(21) Application number: 99959734.7
(22) Date of filing: 09.12.1999
(51) Int. Cl.: A41B 9/02

(54) **THROWAWAY TRUNKS TYPE UNDERPANTS AND METHOD OF PRODUCING THE SAME**

(30) Priority: 10.12.1998 JP 35184498
(71) Applicant: Uni-Charm Company Limited, Kawanoe-Shi, Ehime 799-0111 (JP)
(72) Inventor: KUWASHIRO, Takeo Technical Center, Toyohama-cho Mitoyo-gun Kagawa 769-1602 (JP); SHINOHARA, Tetsushi Technical Center, Toyohama-cho Mitoyo-gun Kagawa 769-1602 (JP); MATSUSHITA, Michiyo Technical Center, Toyohama-cho Mitoyo-gun Kagawa 769-1602 (JP)
(74) Representative: Murgatroyd, Susan Elizabeth
(86) International application number: JP9906925
(87) International publication number: WO0033682

(57) **Abstract**

First and second inner sheets 2, 3 are folded to be up flat together, and sections 2A, 3A of these inner sheets 2, 3 and their both side edges 2c, 3c are folded outwardly of these inner sheets 2, 3. The first inner sheets 2, 2 are bonded together along cutouts 14, 14 of these first inner sheets 2, 2 and the second inner sheets 3, 3 are bonded together along cutouts 14, 14 of these second inner sheets 3, 3. A pair of intermediate sheets 4, 5 are arranged between the first and second inner sheets 2, 3; 2, 3. The pair of intermediate sheets 4, 5 are bonded together along their upper edges. The inner sheets 2, 3 are bonded to the intermediate sheets 4, 5. A pair of outer sheets 6, 7 are placed upon the respective outer sides of the inner sheets 2, 3 and the intermediate sheets 4, 5. The outer sheets 6, 7 are bonded to the inner sheets along the outer side edges 2c, 3c of these inner sheets 2, 3.

## Description

### FIELD OF THE INVENTION

This invention relates to disposable trunks-type pants and a process for making the same.

### BACKGROUND ART

Recently a demand for mass-production of disposable trunks- or brief-type pants has increased as clothes to be conveniently used by travelers or inpatients. However, a high productivity as well as a low manufacturing cost required to meet such a demand can not be achieved by the known usual techniques and steps of the process for making non-disposable pants. As far as the inventors know, none of the techniques meeting the demand has been disclosed in any documents except for the case of disposable diapers. Considering the fact that the disposable pants-type diaper is exclusively used as the undergarment adapted to absorb and contain body wastes, the techniques for making the usual pants-type diaper can not be helpful in making the disposable pants demanded by the travelers or the inpatients.

It is a principal object of this invention to provide pants adapted to be disposable after a single use and a process enabling such pants to be mass-producible at a high productivity per unit time.

### SUMMARY OF THE INVENTION

The object set forth above is achieved, according to an aspect of this invention, by disposable trunks-type pants having a waist-opening and a pair of leg-openings defined by upper and lower edges thereof, respectively, wherein the waist-opening is provided along its peripheral edge with elastic members bonded under tension thereto and extending circumferentially of the pants, the disposable trunks-type pants.

In such pants, this invention is characterized by that the pants comprise a pair of first and second inner sheets each having upper and lower edges, transversely opposite side edges and a cutout formed between the opposite side edges to describe an arc convexly curving from the upper edge toward the lower edge, a pair of intermediate sheets being identical to each other in shape as well as in size, each having upper and lower edges and transversely opposite side edges, and a pair of outer sheets being identical to each other in shape as well as in size, each having upper and lower edges and transversely opposite side edges and provided along the upper edge with the elastic member bonded under tension thereto; the first and second inner sheets respectively have first folding lines extending between the upper and lower edges to bisect respective transverse dimensions between the opposite side edges and second folding lines extending between the upper and lower edges to divide respective transverse dimensions between the first folding lines and the opposite side edges, wherein the first and second inner sheets are respectively folded along the first folding lines to be put flat together, sections defined between the second folding lines and the opposite side edges are folded along the second folding lines outwardly of these inner sheets, the first inner sheets are bonded together along peripheries of the cutouts formed in the first inner sheets and the second inner sheets are bonded together along peripheries of the cutouts formed in the second inner sheets; the intermediate sheets are placed between the first and second inner sheets spaced from each other by a desired distance with the respective cutouts of these intermediate sheets being opposed to each other in such a manner that the lower edges of the first and second inner sheets coincide with the lower edges of the intermediate sheets and the upper edges of the intermediate sheets coincide with bottoms of the cutouts formed in said first and second inner sheets, the intermediate sheets being bonded to each other along the respective upper edges of these intermediate sheets and bonded to the first and second inner sheets along the first folding lines of these inner sheets; and the outer sheets are placed upon outer sides of the first and second inner sheets and the intermediate sheets with the upper and lower edges of the first and second inner sheets and the outer sheets, respectively, and the outer sheets are bonded to the first and second inner sheets along the respective transversely opposite side edges of the first and second inner sheets.

According to one preferred embodiment of this invention, the first and second inner sheets are respectively cut off along the first folding lines of these sheets.

According to another preferred embodiment of this invention, the elastic members are bonded under tension to the outer sheets along the lower edges of the outer sheets.

According to still another embodiment of this invention, the first and second inner sheets and/or the intermediate sheets and/or the outer sheets are made of nonwoven fabric.

The object set forth above is achieved, according to another aspect of this invention, by a process for making disposable trunks-type pants comprising the steps of:
(a) continuously feeding a pair of inner sheets each having a given width forward in the longitudinal direction and placing these inner sheets upon each other in such a manner that respective first center lines of the inner sheets extending in the longitudinal direction to bisect their widths coincide with each other;
(b) bonding the inner sheets thus placed upon each other along first bonding lines formed about the first center lines to curve convexly toward respective opposed side edges of the inner sheets;
(c) cutting out sections of the inner sheets defined inside the first bonding lines of the inner sheets to form the inner sheets with respective apertures;
(d) cutting the inner sheets along the first center lines to divide the inner sheets in first and second inner sheets having outer side edges, inner side edges opposed to each other and cutouts defined by separated halves of the aperture and spacing the first and second inner sheets one from another in the transverse direction with the inner side edges and the cutouts of the first and second inner sheets being opposed one to another, respectively;
(e) continuously feeding a pair of intermediate sheets each having a given width forward in the longitudinal direction and placing said intermediate sheets upon outer sides of the first and second inner sheets in such a manner that respective second center lines extending in the longitudinal direction to bisect their widths coincide with each other and the intermediate sheets lie between the first and second inner sheets arranged to be transversely opposed one to another;
(f) bonding the intermediate sheets to each other along second bonding lines extending transversely of the second center lines inside the first bonding lines of the first and second inner sheets and simultaneously bonding regions of the intermediate sheets defined in the vicinity of their respective side edges to the first and second inner sheets along third bonding lines extending along said inner side edges of the first and second inner sheets;
(g) cutting the intermediate sheets along the second bonding lines to cut off sections of the intermediate sheets extending between the second bonding lines therefrom;
(h) folding the respective outer side edges of the first and second inner sheets along folding lines dividing respective transverse dimensions of these inner sheets between their inner and outer side edges in two, respectively, in such a manner that sections of these inner sheets extending from the folding lines to the outer side edges are folded outwardly of the respective intermediate sheets;
(i) continuously feeding forward a pair of outer sheets in the longitudinal direction, each of the outer sheets having a given width and provided with elastic members extending in the transverse direction and bonded thereto under tension, and placing the outer sheets upon respective outer sides of the first and second inner sheets and the intermediate sheets in such a manner that the second center lines of the intermediate sheets coincide with respective third center lines of the outer sheets extending in the longitudinal direction to bisect respective transverse dimensions of the outer sheets, regions of the first and second inner sheets defined in the vicinity of their outer side edges coincide with regions of the outer sheets defined in the vicinity of their transversely opposite side edges of the outer sheets and the elastic members lie between the second bonding lines of the intermediate sheets;
(j) bonding the regions of the outer sheets defined in the vicinity of their transversely opposite side edges to the first and second inner sheets along fourth bonding lines extending along the respective outer side edges of the first and second inner sheets; and
(k) cutting the first and second inner sheets and the outer sheets along a cutting line extending transversely of these sheets to bisect each of the elastic member bonded to the outer sheets and cutting the first and second inner sheets, the intermediate sheets and the outer sheets along a cutting line extending transversely of these sheets at a position spaced rearward from the cutouts of the first and second inner sheets by a desired distance in the longitudinal direction.

### BRIEF DESCRIPTION OF THE DRAWINGS

Fig. 1 is a perspective view showing trunks-type pants according to this invention;
Fig. 2 is an exploded perspective view of the pants shown in Fig. 1;
Fig. 3 is a plan view of first and second inner sheets;
Fig. 4 is a perspective view showing the partially cutaway pants shown in Fig. 1;
Fig. 5 is a schematic diagram illustrating the steps of the process for making the pants; and
Fig. 6 is a schematic diagram illustrating the steps of the process for making the pants subsequent to those illustrated by Fig. 5.

### DESCRIPTION OF THE BEST MODE FOR WORKING OF THE INVENTION

Disposable trunks-type pants and a process for making the same will be described more in detail by way of example with reference to the accompanying drawings.

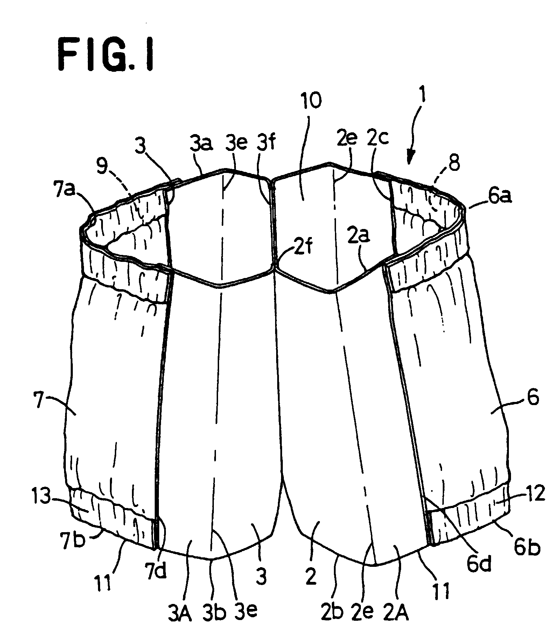

Fig. 1 is a perspective view of disposable trunks-type pants according to a principle of this invention. The pants 1 have a waist-opening 10 defined by upper end thereof to fit elastically around a wearer's trunk and a pair of leg-openings 11 defined by lower end thereof to fit elastically around the wearer's legs. Peripheries of these waist-opening 10 and leg-openings 11 are partially provided with elastic members 8, 9 and 12, 13, respectively, bonded under tension thereto in such a manner that the elastic members 8, 9 circumferentially extend along the wearer's flanks and the elastic members 12, 13 circumferentially extend along outer sides of the wearer's thighs.

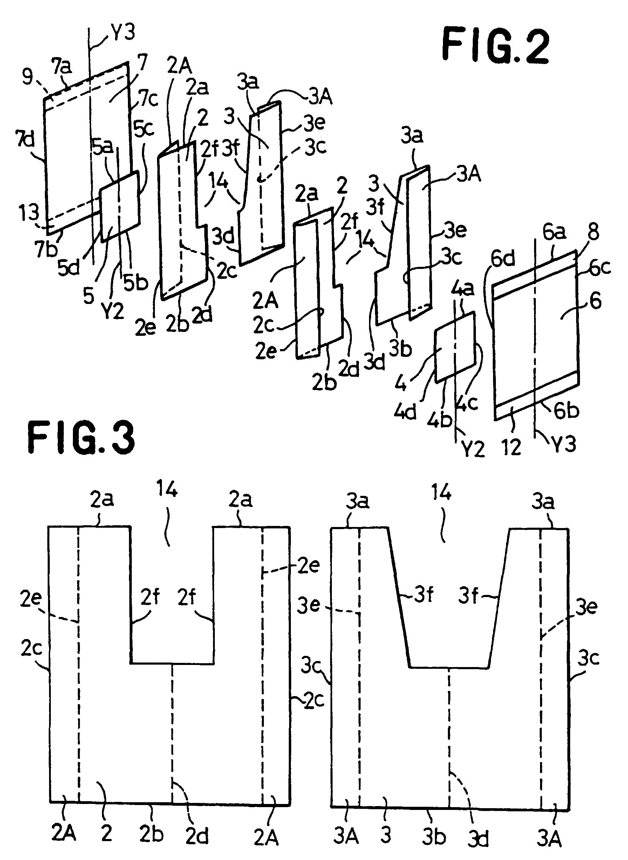

Fig. 2 is an exploded perspective view of the pants shown in Fig. 1 and Fig. 3 is a plan view showing each pair of first and second inner sheets 2, 3. The pants 1 comprise two pairs of first and second inner sheets 2, 3; 2, 3, a pair of intermediate sheets 4, 5 lying outside these pairs of inner sheets 2, 3; 2, 3, and a pair of outer sheets 6, 7 lying outside the pair of intermediate sheets 4, 5.

The pair of first and second inner sheets 2, 3 have upper and lower edges 2a, 2b; 3a, 3b extending in parallel to each other and opposite side edges 2c, 3c extending in parallel to each other transversely of the upper and lower edges 2a, 2b; 3a, 3b. The pair of first and second inner sheets 2, 3 are formed between the respective opposite side edges 2c, 2c; 3c, 3c with cutouts 14 which describe convex arcs extending from the upper edges 2a, 3a toward the lower edges 2b, 3b, respectively. First folding lines 2d, 3d extend between the upper and lower edges 2a, 2b; 3a, 3b of the first and second inner sheets 2, 3 to bisect respective transverse dimensions between their opposite side edges 2c, 2c; 3c, 3c and second folding lines 2e, 3e extend between the respective first folding lines 2d, 3d and the respective side edges 2c, 2c; 3c, 3c.

The first and second inner sheets 2, 3 are folded along the first folding lines 2d, 3d to be put flat together, respectively, and regions 2A, 3A defined between the respective first folding lines 2d, 3d and the respective side edges 2c, 2c; 3c, 3c are folded along the second folding lines 2e, 2e; 3e, 3e outwardly of these inner sheets 2, 3. The first and second inner sheets 2, 3 are arranged to be spaced from each other by a desired distance with their cutouts 14 being opposed to each other. While Fig. 2 illustrates the first and second inner sheets 2, 3 to be cut off from each other along the first folding lines 2d, 3d, it is not essential to cut off them along the first folding lines 2d, 3d.

The intermediate sheets 4, 5 respectively have upper and lower edges 4a, 4b; 5a, 5b extending in parallel to one another and transversely opposite side edges 4c, 4d; 5c, 5d extending in parallel to one another transversely of the upper and lower edges 4a, 4b; 5a, 5b. The intermediate sheets 4, 5 are identical to each other in shape and size and dimensions between their upper and lower edges 4a, 4b; 5a, 5b as well as dimensions between their transversely opposite side edges 4c, 4d; 5c, 5d are smaller than those of the first and second inner sheets 2, 3.

The outer sheets 6, 7 respectively have upper and lower edges 6a, 6b; 7a, 7b extending in parallel to one another and transversely opposite side edges 6c, 6d; 7c, 7d extending in parallel to one another transversely of the upper and lower edges 6a, 6b; 7a, 7b. The outer sheets 6, 7 are identical to each other in shape and size. The outer sheets 6, 7 are provided on their outer surfaces with the elastic members 8, 9 associated with the waist-opening 10 and the elastic members 12, 13 associated with the leg-openings 11. These elastic members 8, 9; 12, 13 are bonded under tension to the outer sheets 6, 7 and extend along the upper and lower edges 6a, 7a; 6b, 7b, respectively. It should be understood that the outer surface of the respective outer sheets 6, 7 may be provided only with said elastic members 8, 9 and the elastic members 12, 13 associated with the leg-openings 11 may be eliminated.

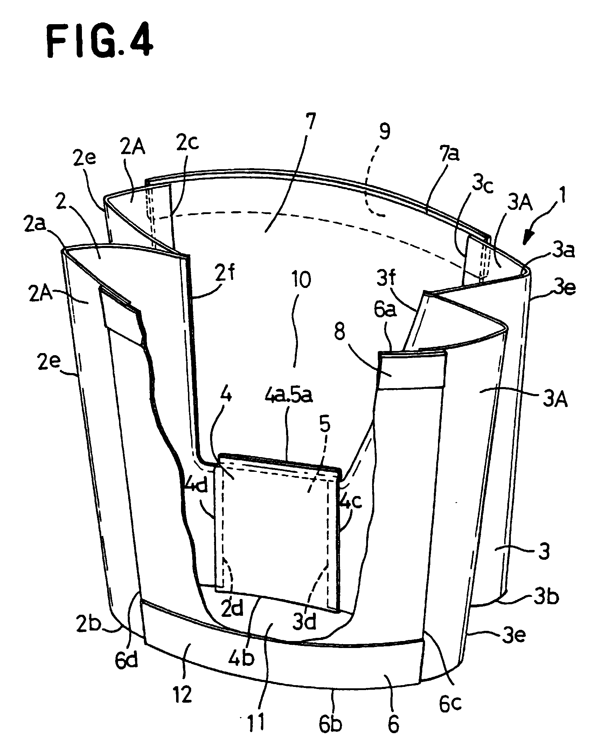

Fig. 4 is a perspective view showing the partially broken away pants 1. The first inner sheets 2 are bonded together along peripheries of their cutouts 14, leaving narrow marginal edges 2f free. Similarly, the second inner sheets 3 are bonded together along peripheries of their cutouts 14, leaving narrow marginal edges 3f free.

The intermediate sheets 4, 5 are interposed between the first and second inner sheets 2, 3 opposed to and spaced from each other with respective centers line Y2 extending between the upper and lower ends 4a, 4b; 5a, 5b to bisect transverse dimensions between the respective opposite side edges 4c, 4d; 5c, 5d of these intermediate sheets 4, 5 being kept in mutual coincidence, on one hand, and the lower edges 2b, 3b; 4b, 5b of the first and second inner sheets 2, 3 and the intermediate sheets 4, 5 being kept in mutual coincidence, on the other hand. The upper edges 4a, 5a of the intermediate sheets 4, 5 lie in coincidence with bottoms of the cutouts 14 formed in the first and second inner sheets 2, 3. The intermediate sheets 4, 5 are bonded together along their upper edges 4a, 5a, leaving these narrow marginal regions of these upper edges 4a, 5a free. The first and second inner sheets 2, 3 and the intermediate sheets 4, 5 are bonded together along the first folding lines 2d, 3d of said first and second inner sheets 2, 3.

The outer sheets 6, 7 are placed upon outer sides of the inner sheets 2, 3 and the intermediate sheets 4, 5 with respective center lines Y3 extending between the upper and lower edges 6a, 6b; 7a, 7b to bisect transverse dimensions between the respective opposite side edges 6c, 6d; 7c, 7d of these outer sheets 6, 7 and the enter lines Y2 of the intermediate sheets 4, 5 being kept in mutual coincidence. In this manner, the first and second inner sheets 2, 3 and the intermediate sheets 4, 5 are disposed between the pair of outer sheets 6, 7. In the vicinity of the respective opposite side edges 6c, 6d; 7c, 7d, the outer sheets 6, 7 are bonded to the first and second inner sheets 2, 3 along their respective opposite side edges 2c, 2c; 3c, 3c.

The first and second inner sheets 2, 3, the intermediate sheets 4, 5 and the outer sheets 6, 7 are preferably made of a spun bond nonwoven fabric or a melt blown nonwoven fabric both formed by thermoplastic fibers. Useful stock material for these sheets further includes a nonwoven fabric made porous to improve its moisture-permeability, a nonwoven fabric embossed to generate irregularity and thereby to improve its cushioning property, and a nonwoven fabric treated to have an elasticity. Taking account of a fact that the first and second inner sheets 2, 3 and the intermediate sheets 4, 5 are destined to be placed against the wearer's crotch region, the stock material having high liquid-absorptivity, moisture-permeability and softness, for example, a nonwoven fabric containing rayon or cotton fibers or a nonwoven fabric resistant against fluffing due to friction is preferably used.

It is possible to adjust a rigidity of the outer sheets 6, 7 to be higher than those of the first and second inner sheets 2, 3 as well as the intermediate sheets 4, 5 for the purpose of stabilizing a shape of the pants 1. For example, a spun bond nonwoven fabric having a basis weight of 40 g/m² or higher may be used as the stock material for the first and second outer sheets 6, 7.

It is also possible to use a nonwoven fabric having an elasticity not only transversely but also longitudinally of the pants 1 as the stock material for the first and second inner sheets 2, 3, the intermediate sheets 4, 5 and the outer sheets 6, 7.

The elastic members 8, 9, 12, 13 may be attached to inner and/or outer surfaces of the outer sheets 6, 7. The elastic members 8, 9, 12, 13 may be formed using material selected from a group consisting of synthetic rubber, natural rubber, elastic film containing synthetic rubber, a spun bond nonwoven fabric or a melt blown nonwoven fabric both containing synthetic rubber as its main ingredient, and composite material such as a laminate of nonwoven fabric and suitable elastic material applied thereon with a tension. In addition, the elastic members 8, 9, 12, 13 may be covered with nonwoven fabric, if desired.

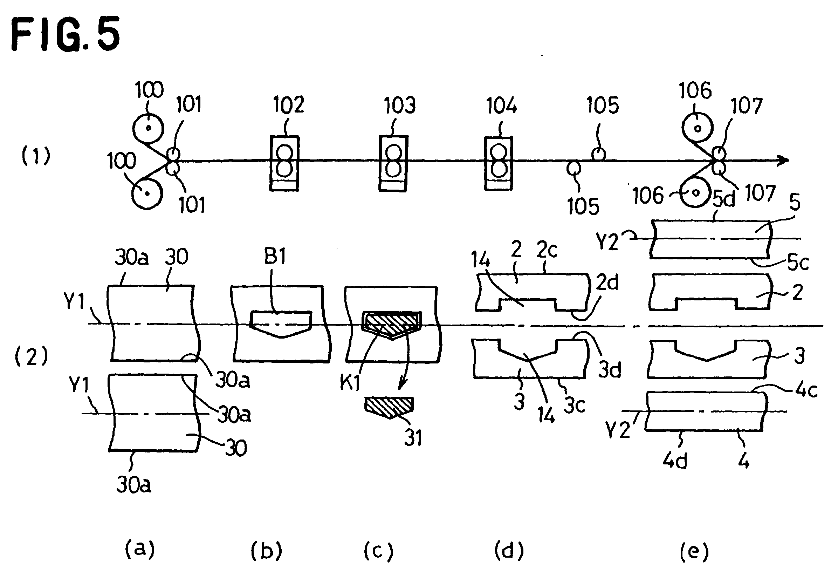

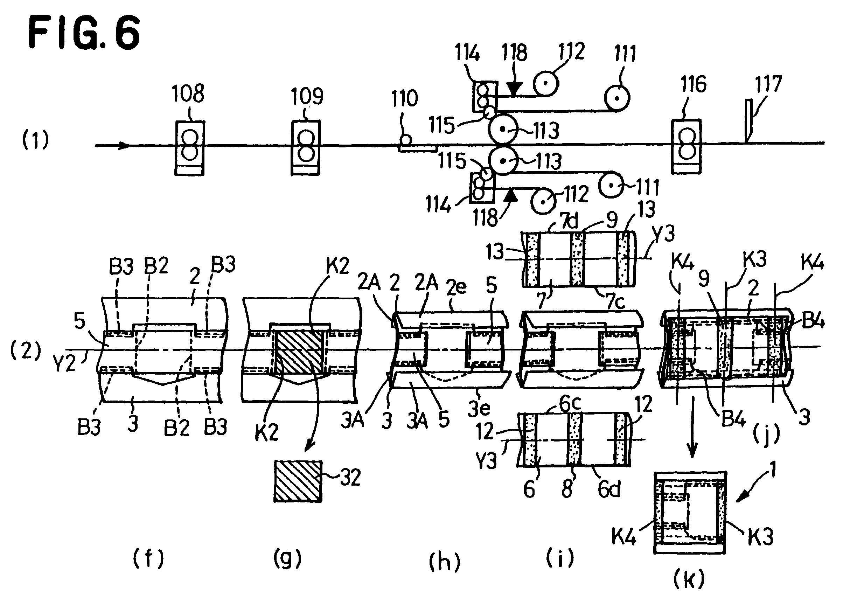

Figs. 5 and 6 are schematic diagrams illustrating, by way of example, sequential the steps of a process for making the pants of this invention, of which Fig. 5 illustrates first step (a) - fifth step (e) and Fig. 6 illustrates sixth step (f) - eleventh step (k) subsequent to the steps illustrated by Fig. 5. of Fig. 5, (1) illustrates the steps in a side view and (2) illustrates the same steps in a plan view. Along a line comprising the first step (a) ∼ the eleventh step (k), the pants 1 are made from a pair of continuous inner sheets 30, 30 both having a given width, a pair of continuous intermediate sheets 4, 5 both having a given width and a pair of continuous outer sheets 6, 7 both having a given width.

The inner sheets 30, 30 are identical to each other in their transverse dimensions and respectively have opposite side edges 30a, 30a extending in the longitudinal direction and center lines Y1, Y1 extending also in the longitudinal direction to bisect the transverse dimensions. The intermediate sheets 4, 5 are identical to each other in their transverse dimensions and respectively have opposite side edges 4c, 4d; 5c, 5d extending in the longitudinal direction and center lines Y2, Y2 extending also in the longitudinal direction to bisect the transverse dimensions. The outer sheets 6, 7 are identical to each other in their transverse dimensions and respectively have opposite side edges 6c, 6d; 7c, 7d extending in the longitudinal direction, center lines Y3, Y3 extending also in the longitudinal direction to bisect the transverse dimensions and film-like elastic members 8, 9, 12, 13 bonded under tension to said outer sheets 6, 7 so as to extend in the transverse direction, respectively.

The sheets 30, 30, 4, 5, 6, 7 are transported through the respective steps (a) ∼ (k) by rotary means such as nip rolls 101, 107 and suction drums 113, 115 adapted to be driven by a driving device.

The first step (a) is a step of placing the inner sheets 30, 30 one upon another. More specifically to describe, the inner sheets 30, 30 stored on respective delivery rolls 100, 100 are continuously fed forward and nipped by a pair of nip rolls 101, 101 located downstream of said delivery rolls 100, 100. The inner sheets 30, 30 are placed one upon another with their center lines Y1, Y1 put in mutual coincidence as these inner sheets 30, 30 pass a pair of nipping surfaces of these rolls 100, 100 and are transported to the second step (b).

The second step (b) is a step of bonding these inner sheets 30, 30 together. Specifically, on the second step (b), the inner sheets 30, 30 placed one upon another are bonded to each other by bonding device 102 along a bonding line B1 describing a closed loop about the center line Y1 which is convex toward the opposite side edges 30a, 30a of the inner sheets 30, 30 exactly placed one upon another.

The third step (c) is a step of forming the inner sheets 30, 30 placed upon each other with an aperture. Specifically, on the third step (c), a cutter device 103 cuts the inner sheets 30, 30 placed upon each other along a cutting line K1 extending along said bonding line B1 and thereby to cut out a portion 31 defined inside said bonding line B1.

The fourth step (d) is a step of cutting the inner sheets 30, 30 to divide them in the first and second inner sheets 2, 3. On this fourth step (d), a cutter device 104 cuts the inner sheets 30, 30 along the center line Y1 in the first and second inner sheets 2, 3. The first and second inner sheets 2, 3 respectively have cutouts 14 defined by the inner edges 2d, 3d transversely opposed to the outer edges 2c, 3c and separated halves of said aperture, respectively. A spacer device 105 transversely spaces the first and second inner sheets 2, 3 from each other by a desired dimension in such a manner that the inner edge 2d and said cutout 14 of the first inner sheet 2 are opposed to those of the second inner sheet 3.

The fifth step (e) is a step of placing the intermediate sheets 4, 5 upon the first and second inner sheets 2, 3, respectively. More specifically to describe, on the fifth step (e), the intermediate sheets 4, 5 stored on a pair of delivery rolls 106, 106 are continuously fed forward. The first and second inner sheets 2, 3 are nipped together with the intermediate sheets 4, 5 by a pair of nip rolls 107, 107 located downstream of said delivery rolls 106, 106. The intermediate sheets 4, 5 are placed upon the respective outer surfaces of the first and second inner sheets 2, 3 with the center lines Y2, Y2 of the intermediate sheets 4, 5 put in mutual coincidence as these sheets 4, 5; 2, 3 pass between nipping surfaces of the nip rolls 107, 107. Then these sheets are transported to the sixth step (f). On this time point, the intermediate sheets 4, 5 are disposed between the first and second inner sheets 2, 3 transversely opposed to each other.

The sixth step (f) is a step of bonding the intermediate sheets 4, 5 to each other and simultaneously bonding these intermediate sheets 4, 5 to the first and second inner sheets 2, 3, respectively. Specifically, on this sixth step (f), a bonding device 108 bonds the intermediate sheets 4, 5 to each other along a pair of bonding lines B2 extending transversely of the center line Y2 inside the bonding line B1 along which the inner sheets 2, 3 are bonded to each other. In the vicinity of the respective transversely opposed side edges 4c, 4d; 5c, 5d, the intermediate sheets 4, 5 are bonded to the inner sheets 2, 3 along bonding lines B3 extending along the inner side edges 2d, 3d of the inner sheets 2, 3.

The seventh step (g) is a step of cutting the intermediate sheets 4, 5 to cut out a portion 32 therefrom. Specifically, on this seventh step (g), a cutter device 109 cuts the intermediate sheets 4, 5 along cutting lines K2 extending along the bonding lines B2 to cut out the portion 32 extending between the bonding lines B2 of the intermediate sheets 4, 5. In this manner, the first and second inner sheets 2, 3 as well as the intermediate sheets 4, 5 are formed with crotch regions.

The eighth step (h) is a step of folding the respective outer side sections 2A, 3A of the first and second inner sheets 2, 3 toward the respective outer sides of the intermediate sheets 4, 5. More specifically, on this eighth step (h), a folder device 110 folds the outer side sections 2A, 2A of the first inner sheets 2, 2 along respective folding lines 2e, 2e extending in the longitudinal direction to bisect respective transverse dimension between the outer and inner edges 2c, 2d of these first inner sheets 2 toward the respective outer sides of the intermediate sheets 4, 5. Similarly, the folder device 110 folds the outer side sections 3A, 3A of the second inner sheets 3, 3 along respective folding lines 3e, 3e extending in the longitudinal direction to bisect respective transverse dimensions between the outer and inner edges 3c, 3d of these second inner sheet 3 toward the respective outer sides of the intermediate sheets 4, 5.

The ninth step (i) is a step of placing the outer sheets 6, 7 upon the outer sides of the first and second inner sheets 2, 3 and the intermediate sheets 4, 5 in such a manner that these sheets 2, 3, 4, 5 are disposed between the pair of outer sheets 6, 7. Specifically to describe, on this ninth step (i), the outer sheets 6, 7 stored on a pair of delivery rolls 111, respectively, are continuously fed forward in the longitudinal direction. The elastic members 8, 9 associated with the waist-opening 10 and the elastic members 12, 13 associated with the leg-openings 11 are transferred onto the outer sheets 6, 7 as these outer sheets 6, 7 travel on peripheral surfaces of a pair of second suction drums 113, 113 located downstream of the respective delivery rolls 111, 111. Then, the outer sheets 6, 7 pass between said pair of second suction drums 113, 113 together with the first and second inner sheets 2, 3 and the intermediate sheets 4, 5. On this step, it is also possible to eliminate the elastic members 12, 13 associated with the leg-openings 11 and to transfer only the elastic members 8, 9 associated with the waist-opening 10 onto the outer sheets 6, 7.

The outer sheets 6, 7 are placed upon the outer sides of the first and second inner sheets 2, 3 and the intermediate sheets 4, 5, respectively, with the center lines Y2, Y2 of the intermediate sheets 4, 5 and the center lines Y3, Y3 of the intermediate sheets 6, 7 put in mutual coincidence as these sheets pass between the contacting surfaces of the pair of suction drums 113, 113. In this course, the first and second inner sheets 2, 3 have their regions in the vicinity of the folded outer edges 2c, 3c put in coincidence with the regions of the outer sheets 6, 7 in the vicinity of their respective opposite side edges 6c, 6d; 7c, 7d. At the same time, the elastic members 8, 9 associated with the waist-opening 10 are placed to transversely extend under tension between the bonding lines B2, B2 of the intermediate sheets 4, 5 and the elastic members 12, 13 associated with the leg-openings 11 are placed to extend across the intermediate sheets 4, 5 which respectively extend between each pair of the adjacent bonding lines B2, B2.

The ninth step (i) is provided with a pair of adhesive applicator device 118 serving to apply the elastic members 8, 9, 12, 13 with adhesive agent and a pair of transfer devices 114 serving to bond these elastic members 8, 9, 12, 13 under tension to the outer sheets 6, 7.

The continuous elastic members stored on a pair of delivery rolls 112, 112 are intermittently applied by the applicator devices 118 with hot melt adhesive. The elastic members applied with adhesive are then transported to the pair of transfer devices 114 in which the elastic members are stretched by suitable stretcher means at a desired draw ratio and successively cut in pieces of a predetermined dimension as measured from respective forward ends. The elastic members 8, 9, 12, 13 thus cut in pieces of the predetermined dimension are then turned by an angle of approximately 90° so as to intersect at right angles the direction in which these elastic members 8, 9, 12, 13 have been transported to this point and held on suction surfaces of the first suction drums 115 as these elastic members are stretched.

The elastic members 8, 9, 12, 13 held on the peripheral surfaces of the first suction drums 115, 115 come in contact with the outer sheets 6, 7 held on the peripheral surfaces of the second suction drums 113 at lines of contact of these suction drums 113, 115; 113, 115. In this way, the elastic members 8, 9, 12, 13 adhesively transferred onto the outer sheets 6, 7 with the desired draw ratio so as to extend transversely of said outer sheets 6, 7.

The tenth step (j) is a step of bonding the outer sheets 6, 7 to the first and second inner sheets 2, 3, respectively. Specifically, on the tenth step (j), a bonding device 116 bonds the regions of the respective outer sheets 6, 7 in the vicinity of their respective opposite side edges 6c, 6d; 7c, 7d to the first and second inner sheets 2, 3 along bonding lines B4 which extend along the outer side edges 2c, 3c of the first and second inner sheets 2, 3, respectively.

The eleventh step (k) is a step of cutting the first and second inner sheets 2, 3, the intermediate sheets 4, 5 and the outer sheets 6, 7 assembled on the precedent steps. On the eleventh step (k), a cutter device 117 cuts the first and second inner sheets 2, 3 and the outer sheets 6, 7 along cutting lines K3 transversely extending to bisect the widths of the elastic members 8, 9 associated with the waist-opening 10. Then, the cutter device 117 cuts the first and second inner sheets 2, 3, the intermediate sheets 4, 5 and the outer sheets 6, 7 along cutting lines K4 transversely extending across these sheets 2, 3, 4, 5, 6, 7 at a desired distance rearward from the cutouts 14 of the first and second inner sheets 2, 3 as viewed in the longitudinal direction so as to bisect the widths of the elastic members 12, 13 associated with the leg-opening 11.

Bonding of the sheets 2, 3, 4, 5, 6, 7 may be carried out using suitable adhesive agent such as hot melt adhesive, or heat- or ultrasonic-sealing technique and cutting of the sheets 2, 3, 4, 5, 6, 7 as well as the elastic members 8, 9, 12, 13 may be carried out using a cutting die or a cutting technique by means of laser or ultrasonic wave.

The production line described heretofore intends to improve a productivity of the pants 1 by continuously running and automating the first step (a) ∼ the eleventh step (k) . The automation may be smoothly achieved by operatively associating the steps (a) ∼ (k) with controller means. For example, a misalignment of the center lines Y1, Y2, Y3 possibly occurring when the sheets 30, 30, 4, 5, 6, 7 are placed one upon another and a deviation possibly occurring in feeding rates of the elastic members 8, 9, 12, 13 and/or the outer sheets 6, 7 from the predetermined feeding rates can be corrected by these controller means operatively associated with the respective steps.

The disposable trunks-type pants according to this invention can be made more easily and less expensively by the process according to this invention than such pants made of stitched cloth pieces. This is for the reason that the pants of this invention can be made merely by bonding the pieces of nonwoven fabric together so far as the sheet of nonwoven fabric is used as the stock material. The pants of this invention satisfactorily meet the requirements for the pants which should be disposable after a single use.

This invention enables a productivity per unit time to be improved by continuously running and automating the steps of the process for making the disposable trunks-type pants.

## Claims

1. Disposable trunks-type pants having a waist-opening and a pair of leg-openings defined by upper and lower edges thereof, respectively, wherein said waist-opening is provided along its peripheral edge with elastic members bonded under tension thereto and extending circumferentially of the pants, said disposable trunks-type pants being characterized by that:
said pants comprise a pair of first and second inner sheets each having upper and lower edges, transversely opposite side edges and a cutout formed between said opposite side edges to describe an arc convexly curving from said upper edge toward said lower edge, a pair of intermediate sheets being identical to each other in shape as well as in size, each having upper and lower edges and transversely opposite side edges, and a pair of outer sheets being identical to each other in shape as well as in size, each having upper and lower edges and transversely opposite side edges and provided along said upper edge with said elastic member bonded under tension thereto;
said first and second inner sheets respectively have first folding lines extending between said upper and lower edges to bisect respective transverse dimensions between said opposite side edges and second folding lines extending between said upper and lower edges to divide respective transverse dimensions between said first folding lines and said opposite side edges, wherein said first and second inner sheets are respectively folded along said first folding lines to be put flat together, sections defined between said second folding lines and said opposite side edges are folded along said second folding lines outwardly of these inner sheets, said first inner sheets are bonded together along peripheries of said cutouts formed in said first inner sheets and said second inner sheets are bonded together along peripheries of said cutouts formed in said second inner sheets;
said intermediate sheets are placed between said first and second inner sheets spaced from each other by a desired distance with the respective cutouts of these intermediate sheets being opposed to each other in such a manner that the lower edges of said first and second inner sheets coincide with the lower edges of said intermediate sheets and the upper edges of said intermediate sheets coincide with bottoms of the cutouts formed in said first and second inner sheets, said intermediate sheets being bonded to each other along the respective upper edges of these intermediate sheets and bonded to said first and second inner sheets along said first folding lines of these inner sheets; and
said outer sheets are placed upon outer sides of said first and second inner sheets and said intermediate sheets with the upper and lower edges of said first and second inner sheets and said outer sheets, respectively, and said outer sheets are bonded to said first and second inner sheets along the respective transversely opposite side edges of said first and second inner sheets.

2. Disposable trunks-type pants according to Claim 1, wherein said first and second inner sheets are respectively cut off along said first folding lines of these sheets.

3. Disposable trunks-type pants according to Claim 1 or 2, wherein said elastic members are bonded under tension to said outer sheets along said lower edges of said outer sheets.

4. Disposable trunks-type pants according to any one of Claims 1 ∼ 3, wherein said first and second inner sheets and/or said intermediate sheets and/or said outer sheets are made of a nonwoven fabric.

5. Process for making disposable trunks-type pants comprising the steps of:
(a) continuously feeding a pair of inner sheets each having a given width forward in the longitudinal direction and placing these inner sheets upon each other so that respective first center lines of said inner sheets extending in said longitudinal direction to bisect their widths coincide with each other;
(b) bonding said inner sheets thus placed upon each other along first bonding lines formed about said first center lines to curve convexly toward respective opposed side edges of said inner sheets;
(c) cutting out sections of said inner sheets defined inside said first bonding lines of said inner sheets to form said inner sheets with respective apertures;
(d) cutting said inner sheets along said first center lines to divide said inner sheets in first and second inner sheets having outer side edges, inner side edges opposed to each other and cutouts defined by separated halves of said aperture and spacing said first and second inner sheets one from another in the transverse direction with said inner side edges and said cutouts of said first and second inner sheets being opposed one to another, respectively;
(e) continuously feeding a pair of intermediate sheets each having a given width forward in said longitudinal direction and placing said intermediate sheets upon outer sides of said first and second inner sheets in such a manner that respective second center lines extending in said longitudinal direction to bisect their widths coincide with each other and said intermediate sheets lie between said first and second inner sheets arranged to be transversely opposed one to another;
(f) bonding said intermediate sheets to each other along second bonding lines extending transversely of said second center lines inside said first bonding lines of said first and second inner sheets and simultaneously bonding regions of said intermediate sheets defined in the vicinity of their respective side edges to said first and second inner sheets along third bonding lines extending along said inner side edges of said first and second inner sheets;
(g) cutting said intermediate sheets along said second bonding lines to cut off sections of said intermediate sheets extending between said second bonding lines therefrom;
(h) folding the respective outer side edges of said first and second inner sheets along folding lines dividing respective transverse dimensions of these inner sheets between their inner and outer side edges in two, respectively, in such a manner that sections of these inner sheets extending from said folding lines to said outer side edges are folded outwardly of the respective intermediate sheets;
(i) continuously feeding forward a pair of outer sheets in said longitudinal direction, each of said outer sheets having a given width and provided with elastic members extending in said transverse direction and bonded thereto under tension, and placing said outer sheets upon respective outer sides of said first and second inner sheets and said intermediate sheets in such a manner that said second center lines of said intermediate sheets coincide with respective third center lines of said outer sheets extending in said longitudinal direction to bisect respective transverse dimensions of said outer sheets, regions of said first and second inner sheets defined in the vicinity of their outer side edges coincide with regions of said outer sheets defined in the vicinity of their transversely opposite side edges of said outer sheets and said elastic members lie between said second bonding lines of said intermediate sheets;
(j) bonding said regions of said outer sheets defined in the vicinity of their transversely opposite side edges to said first and second inner sheets along fourth bonding lines extending along the respective outer side edges of said first and second inner sheets; and
(k) cutting said first and second inner sheets and said outer sheets along a cutting line extending transversely of these sheets to bisect each of said elastic member bonded to said outer sheets and cutting said first and second inner sheets, said intermediate sheets and said outer sheets along a cutting line extending transversely of these sheets at a position spaced rearward from said cutouts of said first and second inner sheets by a desired distance in said longitudinal direction.
